# EUROPÄISCHE PATENTSCHRIFT

(11) **EP 1 261 614 B1**
(45) Veröffentlichungstag und Bekanntmachung des Hinweises auf die Patenterteilung: **07.01.2004**
(21) Anmeldenummer: 01927688.0
(22) Anmeldetag: 22.02.2001
(51) Int. Cl.: C07H 15/00

(54) **VERFAHREN ZUR HERSTELLUNG VON PERBENZYLIERTEN 1-O-GLYCOSIDEN**
METHOD FOR THE PRODUCTION OF PERBENZYLATED 1-O GLYCOSIDES
PROCEDE POUR PRODUIRE DES 1-O-GLYCOSIDES PERBENZYLES

(30) Priorität: 10.03.2000 DE 10013328
(43) Veröffentlichungstag der Anmeldung: 04.12.2002
(73) Patentinhaber: SCHERING AKTIENGESELLSCHAFT, 13342 Berlin (DE)
(72) Erfinder: PLATZEK, Johannes, 12621 Berlin (DE); NIEDBALLA, Ulrich, 14195 Berlin (DE); MARESKI, Peter, 13407 Berlin (DE)
(86) Internationale Anmeldenummer: PCT/EP2001/002024
(87) Internationale Veröffentlichungsnummer: WO 2001/068659

(56) Entgegenhaltungen:
- EP-A- 0 882 733
- US-A- 5 874 411
- SUGAWARA T. ET AL : "Synthesis of (methoxycarbonyl)alkyl and 9-(methoxycarbonyl)-3,6-dixanonyl glycopyranosides for the preparation of carbohydrate glycopyranosides for the preparation of carbohydrate-protein conjugates" CARBOHYDR. RES., Bd. 20, Nr. 1, 1992, Seiten 117-150, XP001018683 in der Anmeldung erwähnt
- SCHMIDT R.R.: "Neue Methoden zur Glycosid- und Oligosaccharidsynthese- gibt es Alternativen zur Koenigs-Knorr-Methode?" ANGEW. CHEMIE, Bd. 98, 1986, Seiten 213-236, XP001018622 in der Anmeldung erwähnt
- SCHMIDT, RICHARD R. ET AL: "O- Alkylation at the anomeric center. Part 5. 1-O- Alkylation of D-glucopyranose" J. CARBOHYDR. CHEM. (1984), 3(1), 67-84 , XP001021740
- LOCKHOFF O.: "An access to glycoconjugate libraries through multicomponent reactions" ANGEW. CHEM. INT. ED., Bd. 37, Nr. 24, 1998, Seiten 3436-3439, XP002171115 in der Anmeldung erwähnt

## Beschreibung

Die Erfindung betrifft ein neues Verfahren zur Herstellung von perbenzylierten 1-O-Glycosiden der allgemeinen Formel I, das in den Patentansprüchen näher gekennzeichnet ist. Das erfindungsgemäße Verfahren geht von kostengünstigen Ausgangsmaterialien aus, liefert gute Ausbeuten und erlaubt die Herstellung von perbenzylierten Sacchariden mit 1-O-funktionalisierten Seitenketten im großen Maßstab.

Perbenzylierte Saccharid-Derivate sind wertvolle Zwischenprodukte in der Synthesechemie. Vor allem die pharmazeutische Chemie verwendet derartige Bausteine sehr häufig, da viele hochpotente und selektive Pharmaka Zuckerreste tragen. So sind beispielsweise im Journal of Drug Targeting 1995, Vol. 3, pp. 111-127 Anwendungen des sogenannten "Glycotargetings" beschrieben. Sogenannte "multi-antennary sugar chains" sind in Chemistry Letters 1998, S. 823 beschrieben. Durch Clustering von Zuckereinheiten wird die Carbohydrat-Rezeptor-Wechselwirkung bei der Zell-Zell-Interaktion wesentlich verbessert. Die Synthese von Galaktosiden mit hoher Affinität zum Asialoglycoprotein Receptor ist im J. Med. Chem. 1995, 38, p. 1538 publiziert worden (siehe auch Int. J. Peptide. Protein Res. 43, 1994, p. 477). Hier werden derivatisierte Galactosen mit funktionalisierten Seitenketten hergestellt, die anschließend an verschiedene andere Moleküle gehängt werden können. Eine gute Übersicht über die Verwendung von Sacchariden als Basis der Glycobiologie ist in Acc. Chem. Res. 1995, 321 gegeben worden . Auch für die Synthese von LewisX Mimetika (Tet. Lett. Vol. 31, 5503) dienen funktionalisierte Monosaccharide als Vorstufen (siehe auch JACS 1996, 118, 6826).

Die Verwendung von derivatisierten Monosacchariden als Zwischenstufen für potentielle Pharmazeutika ist in Current Medicinal Chemistry, 1995, 1, 392 gut dargestellt worden . Perbenzylierte-1-OH-Zucker-Derivate (Galactose, Glucose) werden auch in der Synthese von herzaktiven Glycosiden (Digitoxin-Konjugate) eingesetzt. Die 1-O-Glycosidierung erfolgt hier via Trichloracetimidat und BF₃-Katalyse (J. Med. Chem. 1986, 29, p. 1945). Zur Herstellung immobilisierter Zucker-Liganden (z. B. Verknüpfung an HSA) werden funktionalisierte, geschützte Monosaccharide eingesetzt (Chemical Society Reviews 1995, p. 413).

Ziel einer Gruppe von Synthesen ist es, über eine 1-O-Glycosidierungsreaktion zusätzlich Funktionalität ins Zuckermolekül einzuführen. Hier sind vor allem endständige COOH-, Amino- oder OH-Gruppen von Interesse, da diese in Folgeschritten weiter umgesetzt werden können.

Die Herstellung von 1-O-Glycosiden erfolgt in den meisten Fällen nach klassischen Methoden, wie z.B. nach der von Koenigs-Knorr, Helferich oder der von R.R. Schmidt beschriebenen Trichloracetimidat-Methode [W. Koenigs und E. Knorr, Ber. dtsch. chem. Ges. 34 (1901) 957; B. Helferich u. J. Goendeler, Ber. dtsch. Chem. Ges. 73, (1940) 532; B. Helferich, W. Piel und F. Eckstein, Chem. Ber. 94 (1961), 491; B. Helferich u. W.M. Müller, Chem. Ber. 1970, 103, 3350; G. Wulff, G. Röhle und W. Krüger, Ang. Chem. Internat. Edn., 1970, 9, 455; J. M. Berry u. G.G.S. Duthon, Canad. J. Chem. 1972, 50, 1424; R. R. Schmidt, Angew. Chem. 1986, 98, 213.]

Allen diesen Methoden ist gemeinsam, daß die 1-Hydroxylgruppe in eine reaktive Form überführt wird, die letztlich als Abgangsgruppe dient. Unter Lewissäure-Katalyse (teilweise in stöchiometrischer Menge), erfolgt die eigentliche Umsetzung mit einem Alkohol zum 1-O-Glycosid. Für derartige Umsetzungen gibt es zahlreiche Beispiele in der Literatur.

So ist bei der Herstellung des Immunostimulans KRN-7000 (Kirin Brewery) die Kondensation von Tetra-O-benzyl-β-D-galactopyranosyl-bromid mit einem primären Alkohol, dessen Hydroxylgruppe am Ende einer di-Hydroxy-Amido-C-Kette sitzt (in DMF/Toluol unter Lewissäure Katalyse) ein zentraler Schritt (Drug of the Future 1997, 22(2), p. 185). In dem japanischen Patent JP 95-51764 ist die Umsetzung von 1-O-Acetyl-2,3,4-tri-0-benzyl-L-fucopyranose mit Polyoxyethylen-30-Phytosterol (BPS-30, NIKKO Chem., Japan) unter Trimethylsilylbromid/Zinktriflat-Katalyse beschrieben worden. In Bull. Chem. Soc. 1982, 55(4), p. 1092-6 sind 1-O-Glycosidierungen von Perbenzyl-Zuckern unter Titantetrachlorid-Katalyse in Dichlormethan beschrieben.

In Liebigs Ann. Org. Bioorg. Chem.; EN; 9; 1995; 1673-1680 ist die Herstellung von 3,4,5-Trisbenzyloxy-2-benzyloxymethyl-6-(2-hexadecyloxyethoxy)-tetrahydropyran beschrieben. Ausgehend von 2,3,4,6-Tetra-O-benzyl-D-glucopyranose wird die 1-O-Glycosidierung unter Verwendung von Bu₄NBr, CoBr₂, Me₃SiBr und Molekularsieb in Methylenchlorid innerhalb von 60 Stunden durchgeführt .

Ein Tetrabenzylderivat, das eine endständige, als Methylester geschützte Carboxylgruppe enthält, ist in Carbohydr. Res.; EN; 230; 1; 1992; 117 beschrieben. Die Carboxylgruppe kann danach freigesetzt und weiter umgesetzt werden. Zur Glycosidierung wird Silbercarbonat in Dichlormethan verwendet. Die Verwendung des teuren Silbercarbonats limitiert die Ansatzgröße und macht ein wirtschaftliches up-scaling fast unmöglich. Das gleiche Problem gilt für die nachfolgende Verbindung, die in Tetrahedron Lett. 30, 44, 1989, p. 6019 beschrieben worden ist. Hier wird 2,3,4,6-Tetra-O-benzyl-D-mannosyl-bromid in Nitromethan mit 2-Benzyloxyethanol unter Zurhilfenahme von Quecksilbercyanid zum 1-O-Glycosid umgesetzt. Die Verwendung von Quecksilbercyanid in pilot-plant-Anlagen ist problematisch und aus umweltpolitischer Sicht abzulehnen.

Die in neuester Zeit beschriebenen Substanzbibliotheken für das Hochdurchsatz-Screening verwenden sehr häufig Saccharide (Angew. Chemie 1995, 107, 2912). Hier ist es das Ziel, Zuckerbausteine in geschützter Form vorliegen zu haben, die eine funktionelle Gruppe, wie z.B. -COOH, oder -NH₂ tragen, welche z.B. in einer automatisierten Synthese umgesetzt werden können. Die Bausteine , die dafür Verwendung finden, sind von Lockhoff, Angew. Chem. 1998, 110(24), S. 3634 beschrieben worden. Vor allem die 1-O-Essigsäure von Perbenzyl-Glucose ist hier von Bedeutung. Die Herstellung erfolgt über 2 Stufen, via Trichloracetimidat und Umsetzung mit Hydroxyessigsäureethylester, BF₃-Katalyse in THF und anschließender Verseifung mit NaOH in MeOH/THF. Die Gesamtausbeute über 2 Stufen beträgt allerdings nur 59 %.

Der dabei intermediär durchlaufene 1-O-Essigsäureethylester wird in EP 882733 durch Umsetzen der 2,3,4,6-Tetra-O-benzylglucose mit Hydroxyessigsäureethylester in Gegenwart katalytischer Mengen p-Toluolsulfonsäure durch Kochen in Benzol am Rückfluss erhalten, allerdings ohne Angaben der Ausbeute.

In der gleichen Publikation wird auch die Herstellung eines 1-O-(Aminoethyl)-Glycosids der perbenzylierten Glucose beschrieben. Die Umsetzung erfolgt, wieder ausgehend vom Trichloracetimidat, durch Umsetzung mit N-Formylaminoethanol unter BF₃-Katalyse in THF und anschließender Verseifung in MeOH/THF. Die Gesamtausbeute ist auch hier relativ gering, sie beträgt 45 %.

Ein 1-O-(Aminoethyl)-Derivat der Perbenzylxylose wird in Carbohydrate Research 1997, 298, p. 173 als Zwischenprodukt durchlaufen. Die Synthese ist jedoch sehr langwierig, da sie vom 1-Brom-peracetat der Xylose startet. Die eigentlliche 1-O-Glycosidierung erfolgt über einen 1-Phenylthioether, der mit 2-Azidoethanol unter DMTST-Katalyse (= Dimethyl (methyltio)-sulfonium-triflat) in Dichlormethan umgesetzt wird (Gesamtstufenzahl: 7). Die Gesamtausbeute ist mit unter 40 % für eine industrielle Anwendung nicht geeignet.

Im Übersichtsartikel von R.R. Schmidt in Angew. Chem. 1986, 98, S. 213-236 werden direkte Umsetzungen von 1-OH-Perbenzylglucose und -ribose mit 2-Halogenestern und Triflaten beschrieben. Als Base wird Natriumhydrid in THF oder Benzol verwendet (Chem. Ber. 1982, 115), die Ausbeuten liegen zwischen 40 und 55 %. Auch der Einsatz von Natriumhydrid in Dioxan oder Kalium-tert.-butylat in THF (beides bei Raumtemperatur) ist zur 1-O-Alkylierung mit Triflaten beschrieben (Angew. Chem. 1986, 98, S. 218). Die strengstens einzuhaltenden wasserfreien Reaktionsbedingungen stellen eine große Hürde beim up-scaling derartiger Alkylierungen dar.

Alle bisher bekannten Verfahren haben den großen Nachteil, daß sich ein up-scaling des Prozesses nicht ohne weiteres bewerkstelligen läßt. Die Verwendung von Lewis-Säuren bei der 1-O-Glycosidierung sowie von Natriumhydrid bei der 1-O-Alkylierung erfordert stets wasserfreie Reaktionsbedingungen, was bei großen Ansätzen immer mit Schwierigkeiten verbunden ist. Auch die Aufarbeitung und Entsorgung der Reaktionshilfsstoffe (Hg/Cyanid/etc.) ist in vielen Fällen ein Problem.

Aufgabe der Erfindung war es deshalb, ein Verfahren bereitzustellen, mit dem perbenzylierte Saccharide mit 1-O-funktionalisierter Seitenkette in größerem Maßstab, preiswert und umweltfreundlich hergestellt werden können.

Die Aufgabe der Erfindung wird gemäß dem in den Ansprüchen angegebenen Verfahren gelöst, mit welchem perbenzylierte 1-O-Glycoside der allgemeinen Formel I 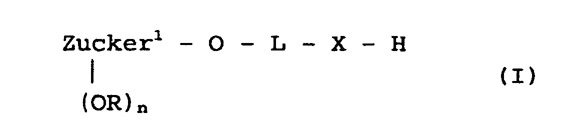 hergestellt werden können. Gemäß Definition der Erfindung bedeutet Zucker¹ in der allgemeinen Formel I ein in 1-OH-Position funktionalisiertes Monosaccharid, wobei es sich hierbei auch um Desoxyzucker handeln kann, die anstelle einer oder mehrerer OH-Gruppen ein H-Atom enthalten. In einer bevorzugten Ausführungsform der Erfindung bedeutet der Zucker in der allgemeinen Formel I ein Monosaccharid mit 5 oder 6 C-Atomen, z. B. Glucose, Mannose, Galactose, Ribose, Arabinose oder Xylose oder deren Desoxyzucker wie beispielsweise 6-Desoxygalactose (Fucose) oder 6-Desoxy-Mannose (Rhamnose).

Der Rest R stellt die Benzylgruppe dar, die in Abhängigkeit vom eingesetzten Monosaccharid oder dessen Desoxyform mindestens zweifach vorhanden ist und beim Einsatz von Di-, Tri- oder Polysacchariden entsprechend mehrfach vorhanden ist.

Der Rest X bedeutet -O-, -S-, -COO- oder -NH-. Im Ergebnis des erfindungsgemäßen Verfahrens werden also Alkohole, Carbonsäuren oder Amine der allgemeinen Formel I erhalten.

Der Rest L kann eine geradkettige, verzweigte, gesättigte oder ungesättigte C₁-C₃₀-Kohlenstoffkette bedeuten, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-, 1-3 Carboxyl-, 1-5 Hydroxy-, 1-5 O-C₁-C₇-alkyl-, 1-3 Aminogruppen, 1-3 CF₃-Gruppen oder 1-10 Fluoratomen. Im Sinne der Erfindung bevorzugte Reste L sind 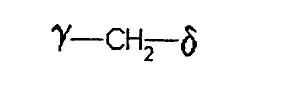 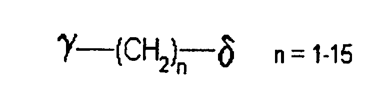 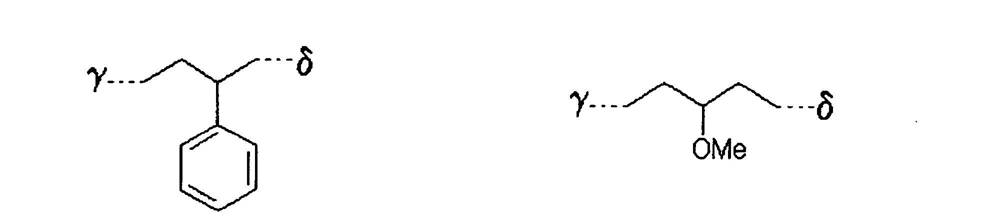 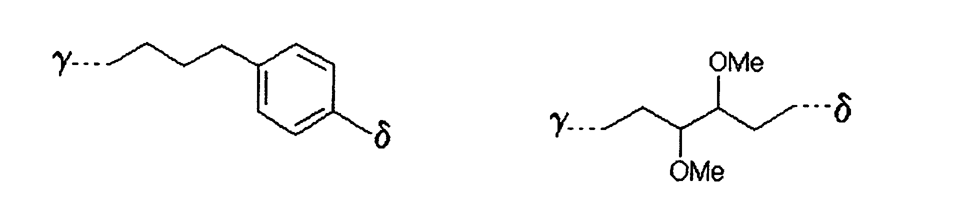 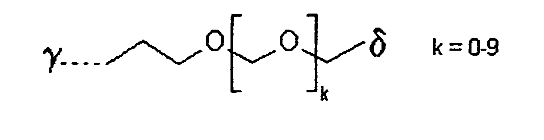 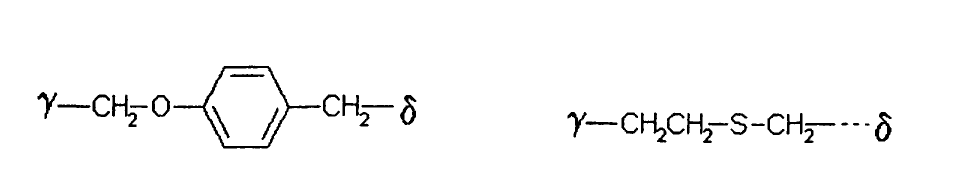 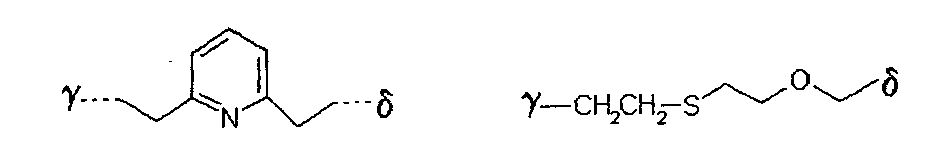 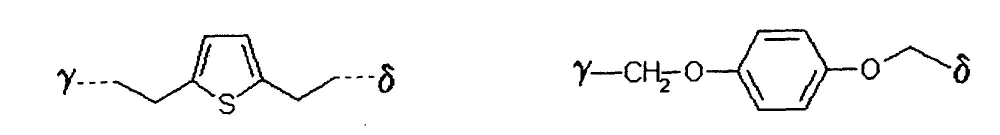 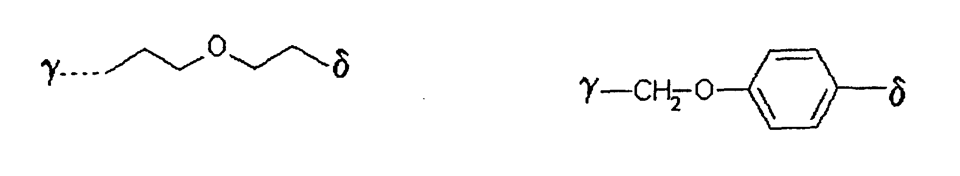 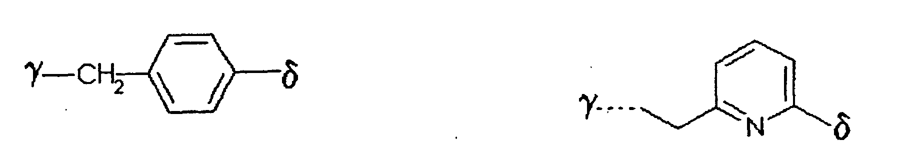 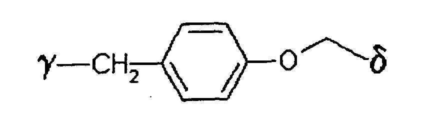 wobei γ die Verknüpfungsstelle am Zucker bedeutet und δ die Verknüpfungsstelle zum Rest X ist. Ein besonders bevorzugter Linker L ist die -CH₂-Gruppe.

Zur Herstellung der perbenzylierten 1-O-Glycoside der allgemeinen Formel I wird ein perbenzylierter 1-OH-Zucker der allgemeinen Formel II 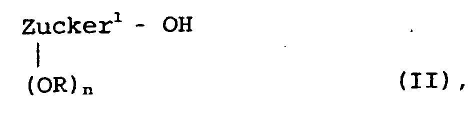 worin Zucker, R und n die oben angegebene Bedeutung haben, in einem mit Wasser nicht mischbaren organischen Lösungsmittel gelöst und mit einem Alkylierungsreagenz der allgemeinen Formel III

Nu - L - X - Sg (III),

worin Nu ein Nucleofug bedeutet, L und X die genannte Bedeutung haben und Sg eine Schutzgruppe ist, in Gegenwart von NaOH oder KOH als Base und gegebenenfalls eines Phasentransfer-Katalysators umgesetzt. Als Nucleofug können im Alkylierungsreagenz der allgemeinen Formel III beispielsweise die Reste -Cl, -Br, -J, -OTs, -OMs, -OSO₂CF₃, -OSO₂C₄F₉ oder -OSO₂C₈F₁₇ enthalten sein.

Bei der Schutzgruppe Sg handelt es sich um eine übliche Säureoder Amin-, Hydroxy- oder Thiol-Schutzgruppe, je nachdem ob X den Rest -O-, -COO- oder -NH- bedeutet. Diese Schutzgruppen sind dem Fachmann gut vertraut (Protective Groups in Organic Syntheses, second Edition, T.W.Greene and P.G.M. Wuts, John Wiley & Sons Inc., New York 1991).

Die erfindungsgemäße Umsetzung kann bei Temperaturen von 0-50°C, vorzugsweise von 0°C bis Raumtemperatur erfolgen. Die Reaktionszeiten betragen von 10 Minuten bis 24 Stunden, vorzugsweise von 20 Minuten bis 12 Stunden.

Die Base wird entweder in fester Form, vorzugsweise fein gepulvert, oder als 10-70%ige, vorzugsweise 30-50%ige, wäßrige Lösung zugesetzt.

Als organische, nicht mit Wasser mischbare Lösungsmittel können im erfindungsgemäßen Alkylierungsverfahren beispielsweise Toluol, Benzol, CF₃-Benzol, Hexan, Cyclohexan, Diethylether, Tetrahydrofuran, Dichlormethan, MTB oder deren Gemische eingesetzt werden.

Als Phasentransfer-Katalysatoren dienen im erf indungsgemäßen Verfahren die für diesen Zweck bekannten quartären Ammoniumoder Phosphoniumsalze oder auch Kronenether wie z. B. [15]-Krone-5- oder [18]-Krone-6. Vorzugsweise kommen quartäre Ammoniumsalze mit vier gleichen oder verschiedenen Kohlenwasserstoffgruppen am Kation, ausgewählt aus Methyl, Ethyl, Propyl, Isopropyl, Butyl oder Isobutyl in Frage. Die Kohlenwasserstoffgruppen am Kation müssen groß genug sein, um eine gute Löslichkeit des Alkylierungsreagenzes im organischen Lösungsmittel zu gewährleisten. Erfindungsgemäß besonders bevorzugt wird N(Butyl)₄⁺-Cl⁻, N(Butyl)₄⁺-HSO₄⁻, aber auch N(Methyl)₄⁺-Cl⁻ eingesetzt.

Nach erfolgter Umsetzung kann die Aufarbeitung des Reaktionsgemisches durch Isolierung des noch geschützten Endproduktes und nachfolgende übliche Abspaltung der Schutzgruppe zum Endprodukt der allgemeinen Formel I erfolgen. Bevorzugt ist es jedoch, das noch geschützte Endprodukt nicht zu isolieren, sondern das Lösungsmittel zu entfernen, den Rückstand in einem neuen, für die Abspaltung der Schutzgruppe geeigneten Lösungsmittel aufzunehmen und hier die Abspaltung durchzuführen. Die Verfahrensweise zur Abspaltung der Schutzgruppe und zur Regenerierung der Säure-, Amino-, Hydroxy- oder Thiolgruppe ist dem Fachmann gut bekannt.

Handelt es sich z. B. bei der Schutzgruppe Sg um eine Säureschutzgruppe, die das acide Proton der Carboxygruppe blockiert, also z. B. um Methyl, Ethyl, Benzyl oder um tert-Butyl, so wird die Säure üblicherweise durch alkalische Hydrolyse regeneriert. In dem Verfahren der Erfindung wird für diesen Fall nach Entfernung des Lösungsmittels aus der Alkylierungsreaktion nun der Rückstand in einem neuen Lösungsmittel, z. B. Methanol, Ethanol, Tetrahydrofuran, Isopropanol, Butanol oder Dioxan aufgenommen. Es wird dann eine wäßrige Lösung einer Base zugesetzt und bei Temperaturen von 0-100°C die alkalische Hydrolyse durchgeführt.

Als Hydroxyschutzgruppen kommen z.B. Benzyl-, 4-Methoxybenzyl-, 4-Nitrobenzyl-, Trityl-, Diphenylmethyl-, Trimethylsilyl-, Dimethyl-tert-butylsilyl oder Diphenyl-tert-butylsilylgruppen in Frage.
Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Fall von Polyolen können die Hydroxygruppen auch in Form von Ketalen mit z. B. Aceton, Acetaldehyd, Cyclohexanon oder Benzaldehyd geschützt sein.
Die Hydroxyschutzgruppen können nach den dem Fachmann bekannten Literaturmethoden, z. B. durch Hydrogenolyse, Säurebehandlung der Ether und Ketale, Alkalibehandlung der Ester oder Behandlung der Silylschutgruppen mit Fluorid freigesetzt werden (siehe z. B. Protective Groups in Organic Syntheses, secon Edition, T.W. Greene and P.G.M. Wuts, John Wiley & Sons, Inc., New Yourk, 1991).
Die Thiolgruppen lassen sich als Benzylether schützen, die mit Natrium in Ammoniak oder siedendem Ethanol spaltbar sind, (W.J. Patterson, v. du Vigneaud, J. Biol. Chem. 111:393, 1993). S-tert-Butylether sind gut mit Fluorwasserstoff/Anisol bei Raumtemperatur spaltbar (S. Salzakibona et al., Bull. Chem. Soc. Japn, 40:2164, (1967)]. S-Benzyloxycarbonylderivate lassen sich durch konzentrierte Ammoniaklösung bei Raumtemperatur bequem spalten (A. Berger et al., J. Am. Chem. Soc., 78:4483, 1956). Erst bei Siedetemperatur werden S-Benzyloxycarbonylderivate von Trifluoressigsäure gespalten [L. Zervas et al, J. Am. Chem. Soc., 85:1337 (1963)].
Die NH₂-Gruppen lassen sich in vielfältiger Weise schützen und wieder freilegen. Das N-Trifluoracetylderivat wird durch Kalium- oder Natriumcarbonat in Wasser [H. Newman, J. Org. Chem., 30:287 (1965), M. A. Schwartz et al., J. Am. Chem. Soc., 95 G12 (1973)] oder einfach durch Ammoniaklösung gespalten [M. Imazama u. F. Eckstein, J. Org. Chem., 44:2039 (1979)]. Ebenfalls milde zu spalten ist das tert-Butyloxycarbonylderivat: es genügt Rühren mit Trifluoressigsäure [B. F. Lundt et al., J. Org. Chem., 43:2285 (1978)]. Sehr groß ist die Gruppe der hydrogenolytisch oder reduzierend zu spaltenden NH₂-Schutzgruppen: Die N-Benzylgruppe ist bequem mit Wasserstoff/Pd-C zu spalten [W.H. Hartung und R. Simonoff, Org. Reactions VII, 263 (1953)], was auch für die Tritylgruppe [L. Zervas, et al., J. Am. Chem. Soc., 78:1359 (1956)] und die Benzyloxycarbonylgruppe gilt [M.Bergmann u. L. Zervas Ber. 65:1192 (1932)].
Von den Silylderivaten werden die leicht spaltbaren tert-Butyldiphenylsilylverbindungen [L. E. Overman et al., Tetrahedron Lett., 27:4391 (1986)], wie auch die 2-(Trimethylsilyl)-ethyl carbamate [L. Grehn et al., Angew. Chem. Int. Ed. Engl., 23:296 (1983)] und die 2-Trimethylsilylethansulfonamide [R.S. Garigipati u. S.M. Weinreb, J. Org. Chem., 53:4134 (1988)] verwendet, die mit Fluoridionen gespalten werden können. Besonders leicht spaltbar ist das 9-Fluorenylmethyl-carbamat: Die Spaltung erfolgt mit Aminen wie Piperidin, Morpholin, 4-Dimethylaminopyridin, aber auch mit Tetrabutylammoniumfluorid [L. A. Corpino et al., J. Org. Chem., 55:1673 (1990); M. Ueki u. M. Amemiya, Tetrahedron Lett., 28:6617(1987)].

Die Isolierung des erhaltenen Endproduktes der allgemeinen Formel I (Alkohol, Thiol, Amin oder Carbonsäure) erfolgt ebenfalls nach üblichen und dem Fachmann gut bekannten Methoden.

So wird beispielsweise im Fall der Säureschutzgruppe das Lösungsmittel aus der Hydrolysereaktion abgedampft und der Rückstand in einem aprotischen Lösungsmittel aufgenommen. Durch Ansäuern mit einer wäßrigen Säurelösung wird der pH auf ca. 2-4 eingestellt und danach die organische Phase abgetrennt. Mittels Kristallisation oder Chromatographie kann nun das perbenzylierte 1-O-Glycosid gewonnen werden.

Gewünschtenfalls können die erhaltenen Verbindungen der allgemeinen Formel I auch in üblicher Weise in ihre Salze überführt werden.

Die Ausbeuten der Verbindungen der allgemeinen Formel I, die mit dem erfindungsgemäßen Verfahren erzielt werden können, sind gut. Sie liegen für bekannte Verbindungen, bei denen ein Vergleich mit dem Stand der Technik möglich ist, über den Ausbeuten des Standes der Technik. So wird beispielsweise für 1-O-Essigsäure von perbenzylierter Glucose eine Gesamtausbeute von 59% in Angew. Chem. 1998, 110 (24), S. 3634 mit dem dort genannten Verfahren beschrieben, während erfindungsgemäß die Ausbeute für diese Verbindung über 2 Stufen 82% beträgt (vgl. Beispiel 7 der vorliegenden Anmeldung). Auch die Herstellung der Verbindung des Beispiels 12 der vorliegenden Anmeldung wird in dieser Publikation beschrieben. Während die Ausbeute an dieser Verbindung erfindungsgemäß 78% über 2 Stufen beträgt, werden mit dem in der Publikation beschriebenen Verfahren lediglich 45% erreicht.

Neben den hohen Ausbeuten bietet das erfindungsgemäße Verfahren auch den Vorteil, daß es von kostengünstigen Startmaterialien ausgeht, ein scale-up des Prozesses ermöglicht und eine leichte Isolierung der Endprodukte erlaubt.

Die Ausgangsmaterialien sind Kaufware oder aus käuflichen Vorstufen leicht erhältlich. So ist bei der Fluka AG, Buchs, Schweiz, die Tetra-2,3,4,6-O-benzyl-D-glucopyranose erhältlich. Bei Fluka sind auch Methyl-D-manno-pyranosid und Methyl-D-galactopyranosid Katalogware. Durch Benzylierung und Spaltung des Glycosids sind 2,3,4,6-Tetra-O-benzyl-D-mannose bzw. -galactose erhältlich.
Über die Sequenz-Methylglycosid-Perbenzyl-methylglycosid-Perbenzyl-1-OH-saccharid lassen sich die Perbenzyl-1-OH-Derivate der Pentosen (Ribose, Arabinose), Hexosen und Desoxyhexosen (Rhamnose, Fucose) gewinnen.

Die erfindungsgemäß hergestellten Verbindungen sind wertvolle Zwischenprodukte in der Synthesechemie. So können sie beispielsweise zum Aufbau von Kohlenhydratdendrimeren, zur Synthese von NMR-Kontrastmitteln und zur Einführung von Zuckerresten in Pharmaka Verwendung finden.

Das erfindungsgemäße Verfahren soll nachfolgend an Ausführungsbeispielen näher erläutert werden.

### Beispiel 1

### 2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,70 g (5 mmol) Tetrabutylammoniumhydragensulfat und 33,7 g (600 mmol) feingepulvertes Kaliumhydroxid in 350 ml Toluol werden auf 0°C abgekühlt. Bei 0°C tropft man 29,3 g (150 mmol) Bromessigsäure tert.butylester über 10 Minuten unter starkem Rühren zu. Man rührt eine Stunde bei 0°C. Man gibt 250 ml MTB (Methyl-tert.butylether) zu, filtriert vom Feststoff ab und dampft das Filtrat im Vakuum zur Trockne ein. Der Rückstand wird in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/nHexan/Ethanol/Essigsäure = 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
50,9 g (85 % d. Th., über 2 Stufen) eines farblosen, zähen Öls

| Elementaranalyse: | | |
|---|---|---|
| ber. | C 72,22 | H 6,40 |
| gef. | C 72,38 | H 6,55 |

### Beispiel 2

### 2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,7 g (5 mmol) Tetrabutylammoniumhydrogensulfat und 24 g (600 mmol) feingepulvertes Natriumhydroxid in 350 ml Toluol werden auf 0°C abgekühlt. Bei 0°C tropft man 29,3 g (150 mmol) Bromessigsäureethylester über 10 Minuten unter starkem Rühren zu. Man rührt eine Stunde bei 0°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
48,5 g (81 % d. Th., über 2 Stufen) eines farblosen, zähen Öls

| Elementaranalyse: | | |
|---|---|---|
| ber. | C 72,22 | H 6,40 |
| gef. | C 72,41 | H 6,61 |

### Beispiel 3

### 2,3,4,6-Tetra-O-benzyl-1-O-carboxymethyl-mannopyranose

Eine Mischung aus 54,1 g (100 mmol) 2,3,4,6-Tetra-O-benzylmannopyranose, 0,55 g (5 mmol) Tetramethylammoniumchlorid und 33,7 g (600 mmol) feingepulvertes Kaliumhydroxid in 350 ml Benzol werden auf 10°C abgekühlt. Bei 10°C tropft man 35,7 g (160 mmol) 6-Bromhexansäureethylester über 10 Minuten unter starkem Rühren zu. Man rührt 2 Stunden bei 10°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
51,7 g (79 % d. Th., über 2 Stufen) eines farblosen Feststoffs

| Elementaranalyse: | | |
|---|---|---|
| ber. | C 73,37 | H 7,08 |
| gef. | C 73,50 | H 7,27 |

### Beispiel 4

### 2,3,4,6-Tetra-O-benzyl-1-O-(1-phenyl-1-carboxy-eth-2-yl)-manopyranose

Eine Mischung aus 54,1 g (100 mmol) 2,3,4,6-Tetra-O-benzylmannopyranose, 1,39 g (5 mmol) Tetrabutylammoniumchlorid und 24 g (600 mmol) feingepulvertes Natriumhydroxid in 350 ml Toluol werden auf 0°C abgekühlt. Bei 0°C tropft man 38,6 g (150 mmol) 2-Phenyl-3-brompropionsäure-ethylester, gelöst in 30 ml Toluol über 10 Minuten unter starkem Rühren zu. Man rührt eine Stunde bei 0°C. Man gibt 250 ml MTB (Methyltert.Butylether) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
54,4 g (79 % d. Th., über 2 Stufen) eines farblosen Feststoffs

| Elementaranalyse: | | |
|---|---|---|
| ber. | C 74,98 | H 6,44 |
| gef. | C 75,11 | H 6,58 |

### Beispiel 5

### 2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,39 g (5 mmol) Tetrabutylammoniumchlorid in 350 ml Toluol und 150 ml 50 %ige aqu. Kali-Lauge werden auf 0°C abgekühlt. Bei 0°C tropft man 30,12 g (200 mmol Chloressigsäure-tert.-butylester über 20 Minuten unter starkem Rühren zu. Man rührt eine Stunde bei 10°C. Man gibt 250 ml Methyl-tert.Butylether zu, trennt die organische Phase ab, extrahiert die wässrige Phase 2 mal mit 250 ml Wasser. Das Lösungsmittel der vereinigten organischen Phasen wird im Vakuum abdestilliert und der Rückstand in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
41,1 g (82 % d. Th., über 2 Stufen) eines farblosen, zähen Öls

| Elementaranalyse: | | |
|---|---|---|
| ber. | C 72,22 | H 6,40 |
| gef. | C 72,01 | H 6,63 |

### Beispiel 6

### 2,3,4,6-Tetra-O-benzyl-1-O-carboxymethyl-glucopyranose

Eine Mischung aus 54,1 g (100 mmol) 2,3,4,6-Tetra-O-benzylglucopyranose 1,39 g (5 mmol) Tetrabutylammoniumchlorid und 24 g (600 mmol) feingepulvertes Natriumhydroxid in 300 ml Tetrahydrofuran werden auf 0°C abgekühlt. Bei 0°C tropft man 78 g (150 mmol) 5-Tosyloxy-pentancarbonsäure-tert.butylester, gelöst in 40 ml Tetrahydrofuran über 30 Minuten unter starkem Rühren zu. Man rührt 3 Stunden bei 0°C. Man gibt 300 ml MTB (Methyl-tert.Butylether) zu, filtriert vom Feststoff ab und dampft das Filtrat im Vakuum zur Trockne ein. Der Rückstand wird in 500 ml Methanol aufgenommen. Man gibt 50 ml 50 %ige aqu. Natronlauge zu und kocht 1 Stunde unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
50 g (78 % d. Th., über 2 Stufen) eines farblosen Feststoffs

| Elementaranalyse: | | |
|---|---|---|
| ber. | C 73,10 | H 6,92 |
| gef. | C 73,21 | H 7,09 |

### Beispiel 7

### 2,3,4,6-Tetra-O-benzyl-1-O-carboxymethyl-glucopyranose

Eine Mischung aus 54,1 g (100 mmol) 2,3,4,6-Tetra-O-benzylglucopyranose, 1,39 g (5 mmol) Tetrabutylammoniumchlorid in 350 ml Toluol und 200 ml 50 %ige aqu. Natron-Lauge wird auf 0°C abgekühlt. Bei 0°C tropft man 29,3 g (150 mmol Bromessigsäure-tert.-butylester über 20 Minuten unter starkem Rühren zu. Man rührt 0,5 Stunden bei 0°C. Man gibt 250 Toluol zu, trennt die organische Phase ab, extrahiert die wässrige Phase 2 mal mit 150 ml Toluol. Das Lösungsmittel der vereinigten organischen Phasen wird im Vakuum abdestilliert und der Rückstand in 400 ml Methanol aufgenommen. Man gibt 50 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Dichlormethan auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n-Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
49,1 g (82 % d. Th., über 2 Stufen) eines farblosen, zähen Öls

| Elementaranalyse: | | |
|---|---|---|
| ber. | C 72,22 | H 6,40 |
| gef. | C 72,09 | H 6,59 |

### Beispiel 8

### 2,3,4,6-Tetra-O-benzyl-1-O-carboxymethyl-glucopyranose

Eine Mischung aus 54,1 g (100 mmol) 2,3,4,6-Tetra-O-benzylglucopyranose, 0,55 g (5 mmol) Tetramethylammoniumchlorid und 33,7 g (600 mmol) feingepulvertes Kaliumhydroxid in 350 ml Benzol werden auf 0°C abgekühlt. Bei 0°C tropft man 44 g (150 mmol) 11-Bromundecansäure-ethylester, gelöst in 50 ml Benzol über 30 Minuten unter starkem Rühren zu. Man rührt 2 Stunden bei 20°C. Man gibt 250 ml Methyl-tert.Butylether zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Dichlormethan auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n-Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
58,4 g (78 % d. Th., über 2 Stufen) eines farblosen Feststoffs

| Elementaranalyse: | | |
|---|---|---|
| ber. | C 75,37 | H 7,54 |
| gef. | C 75,52 | H 7,73 |

### Beispiel 9

### 2,3,4,6-Tetra-O-benzyl-1-O-carboxymethyl-galactopyranose

Eine Mischung aus 54,1 g (100 mmol) 2,3,4,6-Tetra-O-benzylmannopyranose, 1,39 g (5 mmol) Tetrabutylammoniumchlorid in 350 ml Toluol und 150 ml 50 %ige aqu. Kali-Lauge werden auf 0°C abgekühlt. Bei 0°C tropft man 30,12 g (200 mmol Chloressigsäure-tert.-butylester über 20 Minuten unter starkem Rühren zu. Man rührt eine Stunde bei 10°C. Man gibt 250 ml Methyl-tert.Butylether zu, trennt die organische Phase ab, extrahiert die wässrige Phase 2 mal mit 250 ml Wasser. Das Lösungsmittel der vereinigten organischen Phasen wird im Vakuum abdestilliert und der Rückstand in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
41,1 g (82 % d. Th., über 2 Stufen) eines farblosen, zähen Öls

| Elementaranalyse: | | |
|---|---|---|
| ber. | C 72,22 | H 6,40 |
| gef. | C 72,03 | H 6,63 |

### Beispiel 10

### 2,3,4,6-Tetra-O-benzyl-1-O-[1-(4-carboxy)-phenyl-prop-3-yl-galactopyranose

Eine Mischung aus 54,1 g (100 mmol) 2,3,4,6-Tetra-O-benzylgalactopyranose, 1,39 g (5 mmol) Tetrabutylammoniumchlorid und 24 g (600 mmol) feingepulvertes Natriumhydroxid in 300 ml Tetrahydrofuran werden auf 10°C abgekühlt. Bei 10°C tropft man 43 g (150 mmol) 4-(3-Methansulfonyloxy-propyl)-benzoesäureethylester, gelöst in 50 ml Tetrahydrofuran über 30 Minuten unter starkem Rühren zu. Man rührt 2 Stunden bei 10°C. Man gibt 300 ml MTB (Methyl-tert.Butylether) zu, filtriert vom Feststoff ab und dampft das Filtrat im Vakuum zur Trockne ein. Der Rückstand wird in 500 ml Methanol/50 ml Wasser aufgenommen. Man gibt 60 ml 50 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
54,1 g (77 % d. Th., über 2 Stufen) eines farblosen Feststoffs

| Elementaranalyse: | | |
|---|---|---|
| ber. | C 75,19 | H 6,60 |
| gef. | C 75,02 | H 6,79 |

### Beispiel 11

### 2,3,5-Tri-O-benzyl-1-O-carboxymethyl-ribofuranose

Eine Mischung aus 42,1 g (100 mmol) 2,3,5-Tri-O-ribofuranose, 1,39 g (5 mmol) Tetrabutylammoniumchlorid in 350 ml Toluol und 200 ml 50 %ige aqu. Natron-Lauge werden auf 0°C abgekühlt. Bei 0°C tropft man 29,3 g (150 mmol Bromessigsäure-tert.-butylester über 20 Minuten unter starkem Rühren zu. Man rührt eine Stunde bei 0°C. Man gibt 250 ml Methyl-tert.butylether zu, trennt die organische Phase ab, extrahiert die wässrige Phase 2 mal mit 200 ml Methyl-tert.butylether. Das Lösungsmittel der vereinigten organischen Phasen wird im Vakuum abdestilliert und der Rückstand in 500 ml Ethanol aufgenommen. Man gibt 50 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n Hexan/Ethanol/Essigsäure= 20:5:3:0,5). Die produktenthaltenden Fraktionen werden eingedampft, in 200 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
39,2 g (82 % d. Th., über 2 Stufen) eines farblosen, zähen Öls

| Elementaranalyse: | | |
|---|---|---|
| ber. | C 70,28 | H 6,32 |
| gef. | C 70,11 | H 6,51 |

### Beispiel 12

### 2,3,5-Tri-O-benzyl-1-O-(1-amino-eth-2-yl)-ribofuranose

Eine Mischung aus 42,1 g (100 mmol) 2,3,5-Tri-O-benzylribofuranose, 3,40 g (10 mmol) Tetrabutylammoniumhydrogensulfat und 33,7 g (600 mmol) feingepulvertes Kaliumhydroxid in 350 ml Benzol werden auf 10°C abgekühlt. Bei 10°C tropft man 38,1 g (150 mmol)N-(2-Bromethyl)-phthalimid, gelöst in 100 ml Benzol über 40 Minuten unter starkem Rühren zu.. Man rührt 3 Stunden bei 10°C. Man gibt 300 ml Benzol zu, filtriert vom Feststoff ab und dampft das Filtrat im Vakuum zur Trockne ein. Der Filtratrückstand wird in 500 ml Ethanol gelöst, 25,03 g Hydrazinhydrat (500 mmol) zugegeben und 6 Stunden unter Rückfluß erhitzt. Man läßt auf 0°C abkühlen, filtriert vom ausgefallenen Niederschlag ab und dampft das Filtrat im Vakuum zu Trockne ein. Der Rückstand wird in 400 ml Dichlormethan gelöst, diese Lösung 2 mal mit 5 %iger aqu. Natronlauge, anschließend einmal mit Wasser (jeweils 300 ml) gewaschen. Die organische Phase wird im Vakuum zur Trockne eingedampft der Rückstand an Kieselgel chromatographiert (Laufmittel: Dichlormethan/Ethanol/Triethylamin= 20:2:0,1).
Ausbeute:
36,2 g (78 % d. Th., über 2 Stufen) eines farblosen Feststoffs

| Elementaranalyse: | | | |
|---|---|---|---|
| ber. | C 72,55 | H 7,17 | N 3,02 |
| gef. | C 72,39 | H 7,38 | N 2,87 |

### Beispiel 13

### 2,3,4,6-Tetra-O-benzyl-1-O-(1-amino-prop-3-yl)-galactopyranose

Eine Mischung aus 42,1 g (100 mmol) 2,3,4,6-Tetra-O-benzylgalactopyranose, 1,7 g (5 mmol) Tetrabutylammoniumhydrogensulfat und 33,7 g (600 mmol) feingepulvertes Kaliumhydroxid in 350 ml Benzol werden auf 10°C abgekühlt. Bei 10°C tropft man 40,2 g (150 mmol) N-(3-Brompropyl)-phthalimid, gelöst in 100 ml Benzol über 40 Minuten unter starkem Rühren zu. Man rührt 3 Stunden bei 10°C. Man gibt 300 ml Benzol zu, filtriert vom Feststoff ab und dampft das Filtrat im Vakuum zur Trockne ein. Der Filtratrückstand wird in 500 ml Ethanol gelöst, 25,03 ml Hydrazinhydrat (500 mmol) zugegeben und 6 Stunden unter Rückfluß erhitzt. Man läßt auf 0°C abkühlen, filtriert vom ausgefallenen Niederschlag ab und dampft das Filtrat im Vakuum zu Trockne ein. Der Rückstand wird in 400 ml Dichlormethan gelöst, diese Lösung 2 mal mit 5 %iger aqu. Natronlauge, anschließend einmal mit Wasser (jeweils 300 ml) gewaschen. Die organische Phase wird im Vakuum zur Trockne eingedampft , der Rückstand an Kieselgel chromatographiert (Laufmittel: Dichlormethan/Ethanol/Triethylamin= 20:2:0,1).
Ausbeute:
46 g (77 % d. Th. über 2 Stufen) eines farblosen Feststoffs

| Elementaranalyse: | | | |
|---|---|---|---|
| ber. | C 74,35 | H 7,25 | N 2,34 |
| gef. | C 74,24 | H 7,41 | N 2,27 |

### Beispiel 14

### 2,3,4,6-Tetra-O-benzyl-1-O-(1-amino-hex-6-yl)-mannopyranose

Eine Mischung aus 54,1 g (100 mmol) 2,3,4,6-Tetra-O-benzylmannopyranose, 1,39 g (5 mmol) Tetrabutylammoniumchlorid in 350 ml Dichlormethan und 200 ml 60 %ige aqu. Kali-Lauge werden auf 0°C abgekühlt. Bei 0°C tropft man 60,3 g (150 mmol 6-Bromhexylamin-N-(9-fluorenylmethoxy-carbonyl) über 30 Minuten unter starkem Rühren zu. Man rührt eine Stunde bei 0°C. Man gibt 300 ml Dichlormethan zu, trennt die organische Phase ab, extrahiert die wässrige Phase 2 mal mit 200 ml Dichlormethan. Das Lösungsmittel der vereinigten organischen Phasen wird im Vakuum abdestilliert. Der Rückstand wird in 250 ml Ethanol aufgenommen und 100 g (1,17 mol) Piperidin zugegeben. Man rührt 5 Stunden bei 40 °C. Die Lösung wird zur Trockne eingedampft und der Rückstand an Kieselgel chromatographiert (Laufmittel: Dichlormethan/ Ethanol/ Triethylamin= 20:2:0,1).
Ausbeute:
41,1 g (79 % d. Th. über 2 Stufen) eines farblosen Feststoffs

| Elementaranalyse: | | | |
|---|---|---|---|
| ber. | C 69,33 | H 9,50 | N 2,70 |
| gef. | C 69,44 | H 9,68 | N 2,54 |

### Beispiel 15

### 2,3,4-Tri-O-benzyl-6-desoxy-1-O-(1-amino-but-4-yl)-fucopyranose

Eine Mischung aus 43,5 g (100 mmol) 2,3,4-Tri-O-benzyl-6-desoxy-fucopyranose, 1,7 g (5 mmol) Tetrabutylammoniumhydrogensulfat in 350 ml Dichlormethan und 200 ml 60%ige aqu. Natron-Lauge werden auf 0°C abgekühlt. Bei 10°C tropft man 47,4 g (150 mmol) 2-(Trimethylsilyl)-ethylsulfonsäure-N-(4-brombutyl)-amid, gelöst in 100 ml Dichlormethan über 30 Minuten unter starkem Rühren zu. Man rührt 2 Stunden bei 10°C. Man gibt 300 ml Dichlormethan zu, trennt die organische Phase ab, extrahiert die wässrige Phase 2 mal mit 200 ml Dichlormethan. Das Lösungsmittel der vereinigten organischen Phasen wird im Vakuum abdestilliert. Der Rückstand wird in 350 ml Acetonitril aufgenommen und 52,3 g (200 mmol) Tetrabutylammoniumflorid als Monohydrat zugegeben. Man rührt 3 Stunden bei 50 °C. Die Lösung wird zur Trockne eingeengt und der Rückstand an Kieselgel chromatographiert (Laufmittel: Dichlormethan/ Ethanol/ Triethylamin= 20:2:0,1).
Ausbeute:
39,4 g (78 % d. Th. über 2 Stufen) eines farblosen Feststoffs

| Elementaranalyse: | | | |
|---|---|---|---|
| ber. | C 73,64 | H 7,77 | N 2,77 |
| gef. | C 73,53 | H 7,91 | N 2,65 |

### Beispiel 16

### 2,3,4,6-Tetra-O-benzyl-1-0-(3,6,9,12,15-pentaoxa-1-carboxyhexadec-16-yl)-glucopyranose

Eine Mischung aus 54,1 g (100 mmol) 2,3,4,6-Tetra-O-benzylglucopyranose, 1,39 g (5 mmol) Tetrabutylammoniumchlorid und 24 g (600 mmol) feingepulvertes Natriumhydroxid in 350 ml Toluol werden auf 0°C abgekühlt. Bei 0°C tropft man 64,3 g (130 mmol) 17-Tosyloxy-3,6,9,12,15-pentaoxaheptadecansäureethylester, gelöst in 100 ml Tetrahydrofuran über 50 Minuten unter starkem Rühren zu. Man rührt 3 Stunden bei 0°C. Man gibt 300 ml Dichlormethan zu, filtriert vom Feststoff ab und dampft das Filtrat im Vakuum zur Trockne ein. Der Rückstand wird in 500 ml Ethanol/100 ml Wasser aufgenommen. Man gibt 60 ml 60 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 400 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n Hexan/Ethanol/Essigsäure= 20:8:5:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
64,3 g (77 % d. Th., über 2 Stufen) eines farblosen Öls

| Elementaranalyse: | | |
|---|---|---|
| ber. | C 66,17 | H 7,00 |
| gef. | C 66,03 | H 7,19 |

### Beispiel 17

### 2,3,4,6-Tetra-O-benzyl-1-O-(1-hydroxy-eth-2-yl)-mannopyranose

Eine Mischung aus 54,1 g (100 mmol) 2,3,4,6-Tetra-O-benzylmannopyranose, 1,7 g (5 mmol) Tetrabutylammoniumhydrogensulfat und 33,7 g (600 mmol) feingepulvertes Kaliumhydroxid in 350 ml Benzol werden auf 0°C abgekühlt. Bei 0°C tropft man 31,4 g (150 mmol) 2,2-Dimethyl-propionsäure-2bromethylester, über 30 Minuten unter starkem Rühren zu. Man rührt 2 Stunden bei 0°C. Man gibt 300 ml Benzol zu, filtriert vom Feststoff ab und dampft das Filtrat im Vakuum zur Trockne ein. Der Rückstand wird in 500 ml Ethanol/100 ml Wasser aufgenommen. Man gibt 100 ml 50 %ige aqu. Kalilauge zu und kocht 8 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 400 ml Essigsäureethylester auf und stellt unter Rühren den pH-Wert der wässrigen Phase auf pH 5 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/nHexan/ Ethanol= 20:8:2). Die produktenthaltenden Fraktionen werden eingedampft.
Ausbeute:
45,6 g (78 % d. Th., über 2 Stufen) eines farblosen, zähen Öls

| Elementaranalyse: | | |
|---|---|---|
| ber. | C 73,95 | H 6,90 |
| gef. | C 73,84 | H 7,03 |

### Beispiel 18

### 2,3,4,6-Tetra-O-benzyl-1-O-(1-hydroxy-hex-6-yl)-glucopyranose

Eine Mischung aus 54,1 g (100 mmol) 2,3,4,6-Tetra-O-benzylglucopyranose, 1,39 g (5 mmol) Tetrabutylammoniumchlorid und 24 g (600 mmol) feingepulvertes Natriumhydroxid in 350 ml Dichlormethan werden auf 10°C abgekühlt. Bei 10°C tropft man 41,3 g (140 mmol) 1-(Dimethyl-tert.-butylsilyloxy)-6-bromhexan, gelöst in 100 ml Dichlormethan über 50 Minuten unter starkem Rühren zu. Man rührt 3 Stunden bei 10°C. Man gibt 350 ml Dichlormethan zu, filtriert vom Feststoff ab und dampft das Filtrat im Vakuum zur Trockne ein. Der Rückstand wird in 350 ml Acetonitril aufgenommen und 52,3 g (200 ml) Tetrabutylammoniumfluorid (als Monohydrat) zugegeben. Man rührt 3 Stunden bei 50°C. Die Lösung wird zur Trockne eingeengt und der Rückstand an Kieselgel chromatographiert (Laufmittel: Dichlormethan/Ethanol= 20:1).
Ausbeute:
49,3 g (77 % d. Th. über 2 Stufen) eines farblosen Feststoffs

| Elementaranalyse: | | |
|---|---|---|
| ber. | C 74,97 | H 7,55 |
| gef. | C 74,83 | H 7,74 |

### Beispiel 19

### 2,3,4,6-Tetra-O-benzyl-1-O-(1-hydroxy-eth-2-yl)-galactopyranose

Eine Mischung aus 54,1 g (100 mmol) 2,3,4,6-Tetra-O-benzylbenzylgalactopyranose, 1,64 g (15 mmol) Tetramethylammoniumchlorid in 350 ml Toluol und 200 ml 60 %ige aqu. Natron-Lauge werden auf 0°C abgekühlt. Bei 0°C tropft man 52,7 g (130 mmol) 2-(4,4'-Dimethoxytriphenylmethyloxy)-ethylbromid, gelöst in 100 ml Toluol über 30 Minuten unter starkem Rühren zu. Man rührt 3 Stunden bei 0°C. Man gibt 300 ml Toluol zu, trennt die organische Phase ab, extrahiert die wässrige Phase 2 mal mit 200 ml Toluol. Das Lösungsmittel wird im Vakuum abdestilliert. Der Rückstand wird in 500 ml Dichlormethan aufgenommen und 25 g (194 mmol) Dichloressigsäure zugegeben. Man rührt 3 Stunden bei 35 °C. Die Lösung wird 3 mal mit 300 ml 10 %iger aqu. Natronlauge gewaschen und die organische Phase zur Trockne eingeengt und der Rückstand an Kieselgel chromatographiert (Laufmittel: Dichlormethan/ Ethanol= 20:1).
Ausbeute:
46,2 g (79 % d. Th. über 2 Stufen) eines farblosen, zähen Öls

| Elementaranalyse: | | |
|---|---|---|
| ber. | C 73,95 | H 6,90 |
| gef. | C 73, 87 | H 7,05 |

### Beispiel 20

### 2,3,4-Tri-O-benzyl-6-desoxy-1-O-(1-hydroxy-3,6,9,12-tetraazatetradec-14-yl)-galactopyranose

Eine Mischung aus 43,5 g (100 mmol) 2,3,4-Tri-O-benzyl-6-desoxygalactopyranose, 1,39 g (5 mmol) Tetrabutylammoniumchlorid und 24 g (600 mmol) feingepulvertes Natriumhydroxid in 350 ml Tetrahydrofuran werden auf 0°C abgekühlt. Bei 0°C tropft man 66,1 g (130 mmol) 14-Tosyloxy-3,6,9,12-tetraaza-1-(dimethyl-tert.butylsilyloxy)-tetradecan, gelöst in 100 ml Tetrahydrofuan über 40 Minuten unter starkem Rühren zu. Man rührt 3 Stunden bei 10°C. Man gibt 300 ml Dichlormethan zu, filtriert vom Feststoff ab und dampft das Filtrat im Vakuum zur Trockne ein. Der Rückstand wird in 350 ml Acetonitril aufgenommen und 52,3 g (200 ml) Tetrabutylammoniumfluorid als Monohydrat zugegeben. Man rührt 3 Stunden bei 50°C. Die Lösung wird zur Trockne eingeengt und der Rückstand an Kieselgel chromatographiert (Laufmittel: Dichlormethan/ Ethanol= 20:1).
Ausbeute:
51,1 g (78 % d. Th. über 2 Stufen) eines farblosen, zähen Öls

| Elementaranalyse: | | |
|---|---|---|
| ber. | C 67,87 | H 7,70 |
| gef. | C 68,01 | H 7,91 |

## Patentansprüche

1. Verfahren zur Herstellung von perbenzylierten 1-O-Glycosiden der allgemeinen Formel I 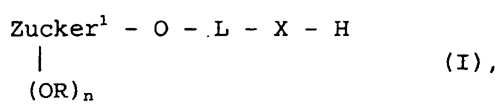 in der
Zucker¹ ein in 1-OH-Position funktionalisiertes Monosaccharid ist,
R Benzyl darstellt,
n 2, 3 oder 4 bedeutet,
X -O-, -S-, -COO- oder -NH- bedeutet
und
L eine geradkettige, verzweigte, gesättigte oder ungesättigte C₁-C₃₀-Kohlenstoffkette bedeutet, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-; 1-3 Carboxyl-, 1-5 Hydroxy-; 1-5 O-C₁-C₇-alkyl-, 1-3 Aminogruppen, 1-3 CF₃-Gruppen oder 1-10 Fluoratomen
oder deren Salzen
**dadurch gekennzeichnet, daß**
ein perbenzylierter 1-OH-Zucker der allgemeinen Formel II 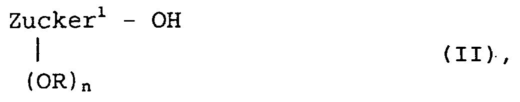 worin Zucker¹, R und n die angegebene Bedeutung haben, mit einem Alkylierungsreagenz der allgemeine Formel (III)
Nu - L - X - Sg (III),
worin Nu ein Nucleofug bedeutet, L und X die genannte Bedeutung haben und Sg eine Schutzgruppe darstellt,
in einem organischen Lösungsmittel in Gegenwart von NaOH oder KOH und gegebenenfalls eines Phasentransfer-Katalysators bei einer Temperatur von 0-50°C umgesetzt wird, anschließend die Schutzgruppe abgespalten wird und das erhaltene Reaktionsprodukt gegebenenfalls in ein Salz überführt wird.

2. Verfahren nach Anspruch 1,
**dadurch gekennzeichnet, daß**
als perbenzylierter 1-OH-Zucker der allgemeinen Formel II ein perbenzyliertes Monosaccharid mit 5 bis 6 C-Atomen oder dessen Desoxy-Verbindung eingesetzt wird.

3. Verfahren nach Anspruch 1 oder 2,
**dadurch gekennzeichnet, daß**
als perbenzylierter 1-OH-Zucker der allgemeinen Formel II perbenzylierte Glucose, Mannose, Galactose, Ribose, Arabinose, Xylose, Fucose oder Rhamnose eingesetzt wird.

4. Verfahren nach einem der Ansprüche 1 bis 3,
**dadurch gekennzeichnet, daß**
als Alkylierungsreagenz der allgemeinen Formel III ein solches eingesetzt wird, in welchem das Nucleofug die Reste -Cl, -Br, -J, -OTs, -OMs, -OSO₂CF₃, -OSO₂C₄F₉ oder -OSO₂C₈F₁₇ bedeutet.

5. Verfahren nach einem der Ansprüche 1 bis 4,
**dadurch gekennzeichnet, daß**
als Alkylierungsreagenz der allgemeinen Formel III ein solches eingesetzt wird, in welchem der Rest L 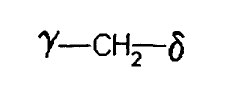 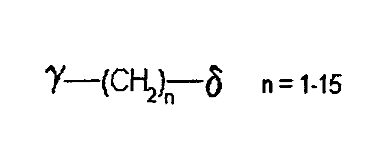 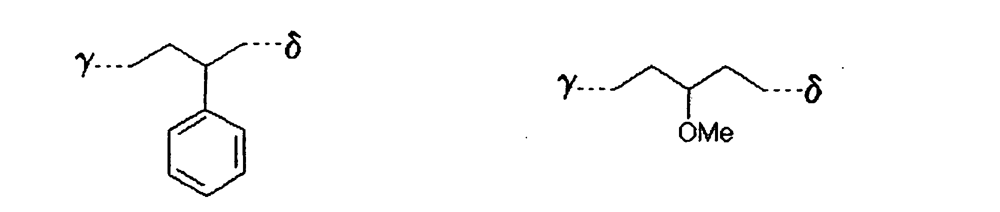 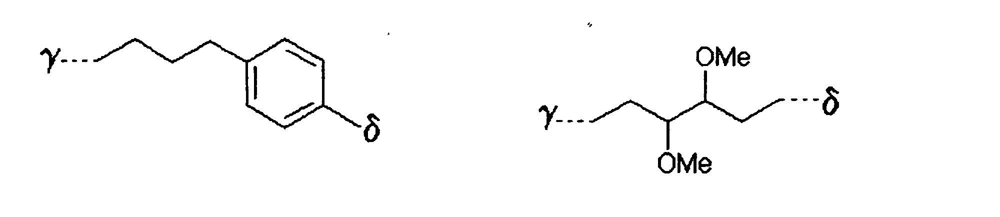 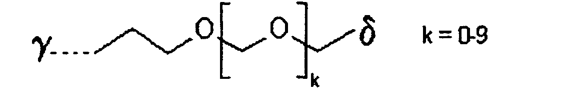 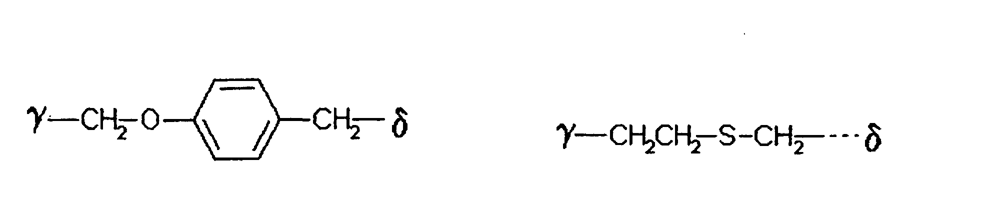 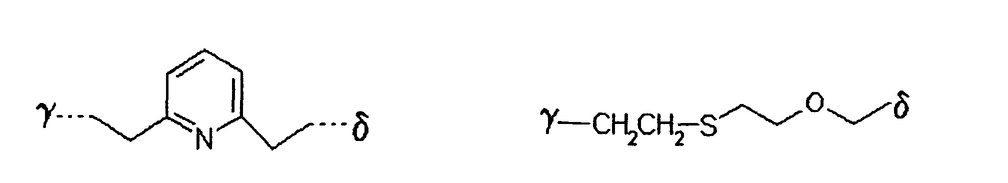 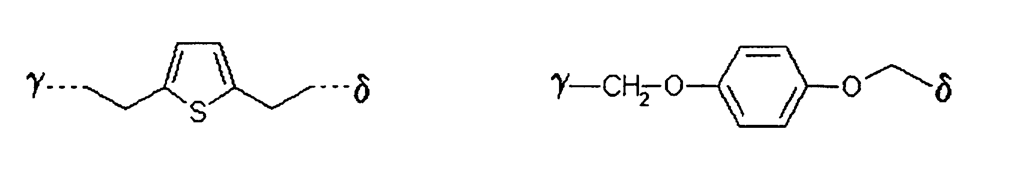 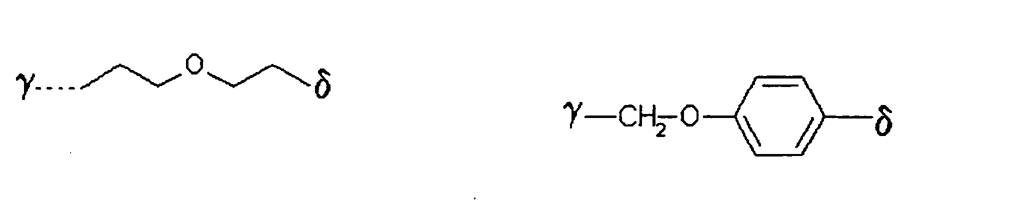 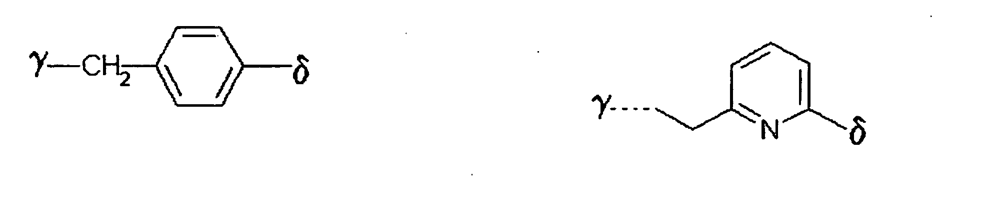 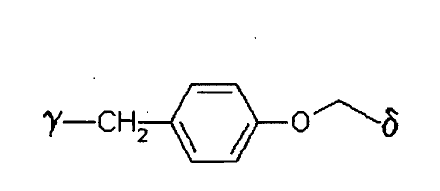 bedeutet,
wobei γ die Verknüpfungsstelle am Zucker bedeutet und δ die Verknüpfungsstelle zum Rest X ist.

6. Verfahren nach einem der Ansprüche 1 bis 5,
**dadurch gekennzeichnet, daß**
als organisches Lösungsmittel ein mit Wasser nicht mischbares Lösungsmitel eingesetzt wird, vorzugsweise Toluol, Benzol, CF₃-Benzol, Hexan, Cyclohexan, Diethylether, Tetrahydrofuran, Dichlormethan, MTB oder deren Gemische.

7. Verfahren nach einem der Ansprüche 1 bis 6,
**dadurch gekennzeichnet, daß**
als Phasentransfer-Katalysator ein quartäres Ammonium- oder Phosphoniumsalz oder ein Kronenether eingesetzt wird, vorzugsweise ein quartäres Ammoniumsalz.

8. Verfahren nach einem der Ansprüche 1 bis 7,
**dadurch gekennzeichnet, daß** NaOH oder KOH in fester Form oder als 10-70%ige wäßrige Lösung zugesetzt wird.

## Claims

1. Process for the production of perbenzylated 1-O-glycosides of general formula I 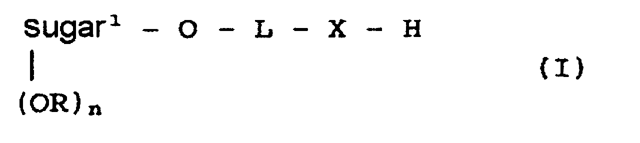 in which
sugar¹ is a monosaccharide that is functionalized in 1-OH- position,
R represents benzyl,
n means 2, 3 or 4,
X means-O-,-S-,-COO- or -NH-
and
L means a straight-chain, branched, saturated or unsaturated C₁-C₃₀ carbon chain, which optionally is interrupted by 1-10 oxygen atoms, 1-3 sulphur atoms, 1-2 phenylene groups, 1-2 phenyleneoxy groups, 1-2 phenylenedioxy groups, a thiophene radical, pyrimidine radical or pyridine radical, and/or optionally is substituted with 1-3 phenyl groups, 1-3 carboxyl groups, 1-5 hydroxy groups, 1-5 O-C₁-C₇ alkyl groups, 1-3 amino groups, 1-3 CF₃ groups or 1-10 fluorine atoms
of salts thereof,
**characterized in that** it comprises reacting
a perbenzylated 1-OH-sugar of general formula II 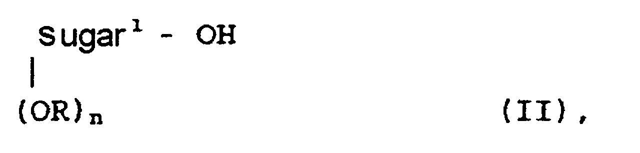 in which sugar¹, R and n have the indicated meaning,
with an alkylating reagent of general formula (III)
Nu - L - X - Sg (III),
in which Nu means a nucleofuge, L and X have the above-mentioned meaning, and Sg represents a protective group,
in an organic solvent in the presence of NaOH or KOH and optionally a phase transfer catalyst at a temperature of 0-50°C, then detaching the protective group, and optionally converting the reaction product obtained into a salt.

2. Process according to Claim 12, **characterized in that** a perbenzylated monosaccharide with 5 to 6 C-atoms or a deoxy compound thereof is used as a per benzylated 1-OH-sugar of general formula II.

3. Process according to Claim 1 or 2, **characterized in that** perbenzylated glucose, mannose, galactose, ribose, arabinose, xylose, fucose or rhamnose is used as a perbenzylated 1-OH-sugar of general formula II.

4. Process according to one of Claims 1 to 3, **characterized in that** the alkylating reagent of general formula III which is used is one in which the nucleofuge is -Cl, -Br, -I, -OTs, -OMs, -OSO₂CF₃, -OSO₂C₄F₉ or -OSO₂C₈F₁₇.

5. Process according to any one of Claims 1 to 4, **characterized in that** the alkylating reagent of general formula III which is used is one in which L means 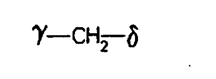 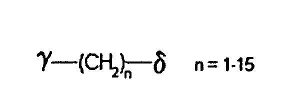 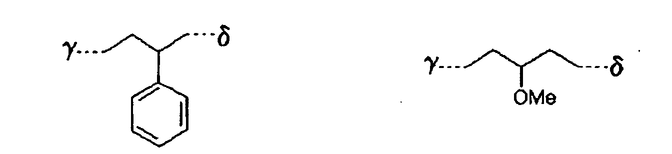 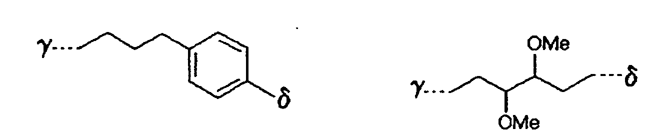 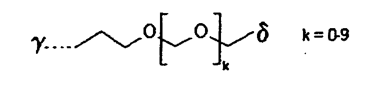 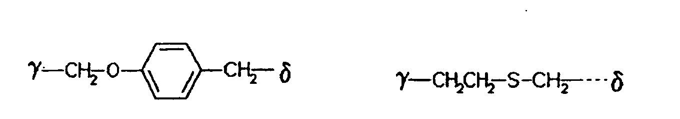 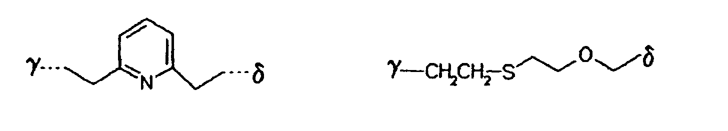 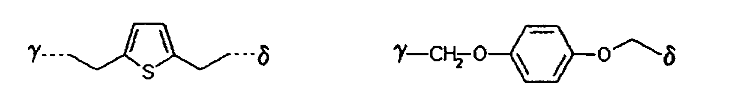 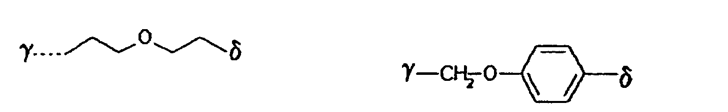 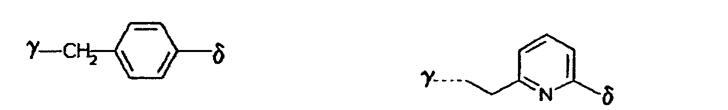 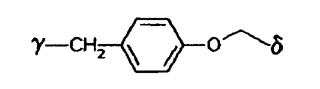 where γ is the interface site to sugar, and is the interface site to radical X.

6. Process according to any one of Claims 1 to 5, **characterized in that** a water-immiscible solvent, preferably toluene, benzene, CF₃-benzene, hexane, cyclohexane, diethyl ether, tetrahydrofuran, dichloromethane, MTB or mixtures thereof, is used as an organic solvent.

7. Process according to any one of Claims 1 to 6, **characterized in that** a quaternary ammonium or phosphonium salt or a crown ether, preferably a quaternary ammonium salt, is used as a phase transfer catalyst.

8. Process according to any one of Claims 1 to 7, **characterized in that** NaOH or KOH is added in solid form or as a 10-70% aqueous solution.

## Revendications

1. Procédé pour la préparation de 1-O-glycosides perbenzylés de formule générale I 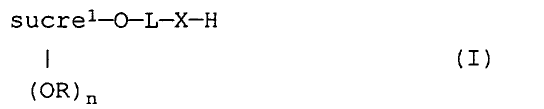 dans laquelle
sucre¹ est un monosaccharide fonctionnalisé en position 1-OH,
R représente le groupe benzyle,
n représente 2, 3 ou 4,
X représente -O-, -S-, -COO- ou -NH- et
L représente une chaîne carbonée en C₁-C₃₀ droite, ramifiée, saturée ou insaturée, qui est éventuellement interrompue par 1-10 atomes d'oxygène, 1-3 atomes de soufre, 1-2 groupes phénylène, 1-2 groupes phénylène-oxy, 1-2 groupes phénylènedioxy, un radical thiophène, pyrimidine ou pyridine, et/ou est éventuellement substituée par 1-3 groupes phényle, 1-3 groupes carboxy, 1-5 groupes hydroxy, 1-5 groupes O-alkyle(C₁-C₇), 1-3 groupes amino, 1-3 groupes CF₃ ou 1-10 atomes de fluor,
ou de leurs sels,
**caractérisé en ce qu'**on fait réagir un 1-OH-sucre perbenzylé de formule générale II 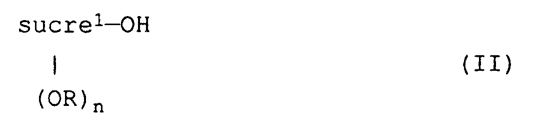 dans laquelle sucre¹, R et n ont les significations indiquées,
avec un réactif d'alkylation de formule générale (III)
Nu-L-X-Sg (III)
dans laquelle Nu représente un nucléofuge, L et X ont les significations données et Sg représente un groupe protecteur
dans un solvant organique en présence de NaOH ou KOH et éventuellement d'un catalyseur de transfert de phase, à une température de 0-50°C, on élimine ensuite le groupe protecteur et le produit de réaction obtenu est éventuellement converti en un sel.

2. Procédé selon la revendication 1, **caractérisé en ce qu'**on utilise en tant que 1-OH-sucre perbenzylé de formule générale II un monosaccharide perbenzylé ayant 5 ou 6 atomes de carbone ou un composé désoxy de celui-ci.

3. Procédé selon la revendication 1 ou 2, **caractérisé en ce qu'**on utilise en tant que 1-OH-sucre perbenzylé de formule générale II du glucose, mannose, galactose, ribose, arabinose, xylose, fucose ou rhamnose perbenzylé.

4. Procédé selon l'une quelconque des revendications 1 à 3, **caractérisé en ce qu'**on utilise en tant que réactif d'alkylation de formule générale III un tel réactif dans lequel le nucléofuge représente les radicaux -Cl, - Br, -I, -OTs, -OMs, -OSO₂CF₃, -OSO₂C₄F₉ ou -OSO₂C₈F₁₇.

5. Procédé selon l'une quelconque des revendications 1 à 4, **caractérisé en ce qu'**on utilise en tant que réactif d'alkylation de formule générale III un tel réactif dans lequel le radical L représente 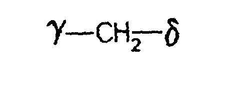 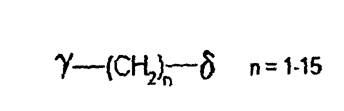 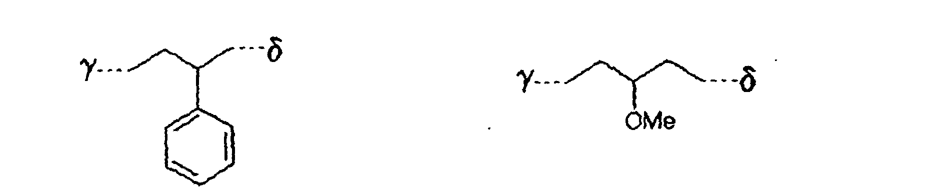 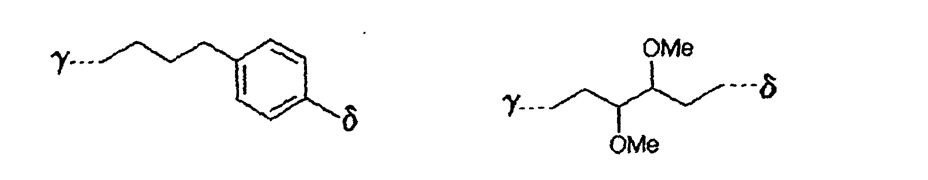 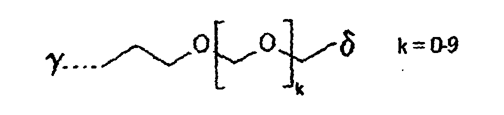 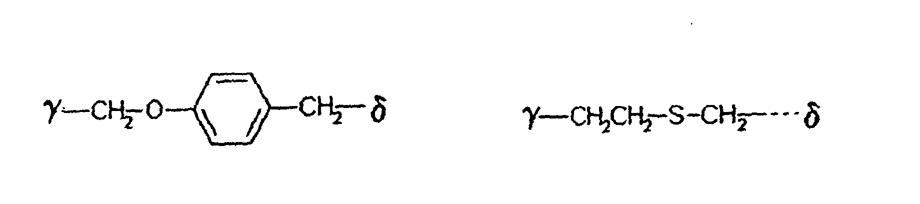 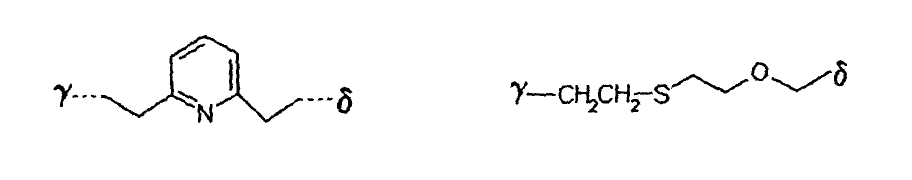 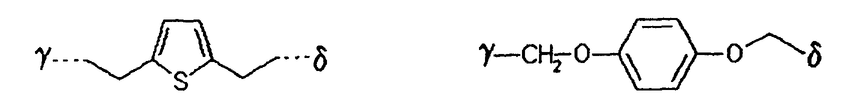 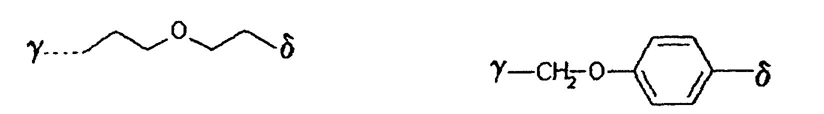 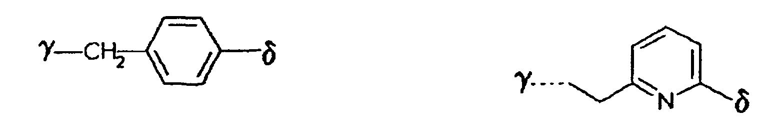 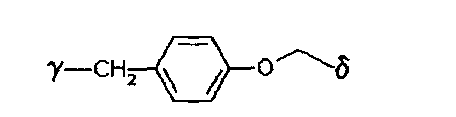 γ représentant le point d'attachement au sucre et δ étant le point d'attachement au radical X.

6. Procédé selon l'une quelconque des revendications 1 à 5, **caractérisé en ce qu'**on utilise en tant que solvant organique un solvant non miscible à l'eau, de préférence le toluène, le benzène, le CF₃-benzène, l'hexane, le cyclohexane, l'oxyde d'éthyle, le tétrahydrofuranne, le dichlorométhane, le MTB (oxyde de méthyle et de tert-butyle) ou des mélanges de ceux-ci.

7. Procédé selon l'une quelconque des revendications 1 à 6, **caractérisé en ce qu'**on utilise en tant que catalyseur de transfert de phase un sel d'ammonium ou phosphonium quaternaire ou un éther-couronne, de préférence un sel d'ammonium quaternaire.

8. Procédé selon l'une quelconque des revendications 1 à 7, **caractérisé en ce que** NaOH ou KOH est ajouté sous forme solide ou sous forme de solution aqueuse à 10-70 %.
